# EUROPEAN PATENT APPLICATION

(11) **EP 4 582 418 A1**
(43) Date of publication of application: **09.07.2025**
(21) Application number: 23859322.2
(22) Date of filing: 29.08.2023
(51) Int. Cl.: C07D 401/14, C07D 491/056, A61K 31/4709, A61K 31/501, A61K 31/506, A61P 35/00, A61P 15/00

(54) **CRYSTALLINE FORM OF QUINOLINE DERIVATIVE INHIBITOR, PREPARATION METHOD THEREFOR AND USE THEREOF**

(30) Priority: 30.08.2022 CN 202211052009
(71) Applicant: TransThera Sciences (Nanjing), Inc., Nanjing, Jiangsu 210032 (CN)
(72) Inventor: KANG, Sishun, Nanjing, Jiangsu 210032 (CN); YUAN, Jiadong, Nanjing, Jiangsu 210032 (CN); LI, Lin, Nanjing, Jiangsu 210032 (CN)
(74) Representative: Ström & Gulliksson AB
(86) International application number: PCT/CN2023/115393
(87) International publication number: WO 2024/046292

(57) **Abstract**

The present invention relates to the technical field of medicines, and in particular, to a mesylate crystalline form of a TAM family kinase and/or CSF1R kinase and/or NTRK inhibitor shown in a formula (I), a preparation method therefor and a pharmaceutical use thereof.

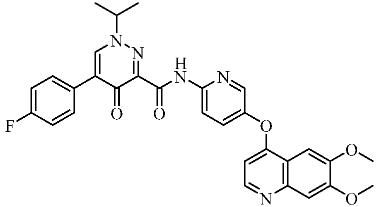

## Description

### CROSS-REFERENCE TO RELATED APPLICATION

The present disclosure claims priority to the invention patent application No. 202211052009.8 filed with China National Intellectual Property Administration on August 30, 2022 and entitled "CRYSTALLINE FORM OF QUINOLINE DERIVATIVE INHIBITOR, PREPARATION METHOD THEREFOR AND USE THEREOF", which is incorporated herein by reference in its entirety.

### TECHNICAL FIELD

The present disclosure relates to the technical field of pharmaceuticals, and particularly relates to a crystal form of a TAM family kinase and/or CSF1R kinase inhibitor, a preparation method therefor, and pharmaceutical use thereof. The crystal form of the present disclosure can selectively inhibit the tyrosine kinase TAM family and/or the CSF1R kinase, and can be used for treating and/or preventing a disease mediated by the abnormal expression of a TAM family kinase receptor and/or CSF1R kinase receptor and/or a ligand thereof. Furthermore, the crystal form of the present disclosure can also be used for treating and/or preventing an NTRK-caused related disease, and more specifically can be used for treating and/or preventing a related disease of drug-resistant type resulting from mutation of NTRK.

### BACKGROUND

The TAM family includes three members of Axl, Mer, and Tyro-3, and contains an extracellular domain, a transmembrane domain, and a conserved intracellular kinase domain. Axl (also referred to as UFO, Ark, Tyro-7, or JTK1), Mer (also referred to as c-Mer, Mertk, Eyk, Nyk, or Tyro-12), Tyro-3 (also referred to as Sky, Byk, Rse, Dtk, etc), galectin-3, Gas6, and ProS are abnormally expressed in various solid tumors, such as lung cancer, gastric cancer, and liver cancer, and in various hematological tumors, such as AML, ALL, and CML, and are closely related to poor prognosis of a disease, disease progression, tumor metastasis, tumor drug resistance, etc. (Douglas K, Nature Reviews, 2014). In particular, Axl, as a tyrosine kinase, has been proved to be one of the causes of drug resistance to EGFR inhibitors in NSCLC, and is closely related to the metastasis of various solid tumors.

The colony stimulating factor 1 receptor (CSF1R), also referred to as c-FMS, FMS, FIM2, MCSF, and CD115, is a single-chain transmembrane glycoprotein composed of 972 amino acid residues, which belongs to the type III receptor tyrosine kinase (RTK) family, together with FLT-3, PDGFR, and KIT. In the CSF1/CSF1R pathway, the signal axis formed by CSF1 and CSF1R can promote the growth of macrophages in the body while regulating the normal development and homeostasis of tissues and organs, and homeostatic imbalance can cause a variety of diseases, such as inflammation, immune system diseases, and tumors.

NTRK genes include NTRK1, NTRK2, and NTRK3, which are responsible for the synthesis of tropomyosin-related kinase (TRK) family proteins, TRKA, TRKB, and TRKC, respectively. NTRK gene fusion can occur anywhere in the body and has been found in a variety of solid tumors in adults and children, including mammary analog secretory carcinoma (MASC), colon cancer, lung cancer, pancreatic cancer, thyroid cancer, and various sarcomas. NTRK fusion occurs at a very low frequency in common cancer types such as colon cancer and lung cancer, with both accounting for less than 5%; however, in certain rare cancer types such as congenital renal tumor, infantile sarcoma, salivary gland carcinoma, and secretory breast carcinoma, the mutation rates are extremely high, with some cancer types having a frequency greater than 90% (Journal of Clinical Oncology, 2017, Volume 15, Issue 12). Therefore, the development of NTRK inhibitors has great clinical value.

In the R&D process of drugs, researches on crystal forms are very important. Compared with other forms, the crystal forms of compounds are very different in terms of stability, solubility, and the like. WO2020038460A1 discloses compounds as inhibitors of the TAM family kinases and/or CSF1R kinase and/or NTRK, which can be used for treating and/or preventing diseases mediated by the abnormal expression of TAM family kinase receptors and/or CSF1R kinase receptors and/or ligands thereof, including tumor, immune dysregulation, endometriosis, vascular disease/trauma, psoriasis, visual defect/lesion (caused by macular degeneration, diabetes, premature delivery, etc.), kidney disease, rheumatoid arthritis, osteoporosis, and other related diseases. Furthermore, these compounds can also be used in the manufacture of medicaments for treating and/or preventing NTRK-caused related diseases, especially related diseases of drug-resistant type resulting from mutation of NTRK, wherein the NTRK-caused related diseases include, but are not limited to, non-small cell lung cancer, colorectal cancer, mammary analog secretory carcinoma, sarcoma, astrocytoma, glioblastoma, Spitz-like melanoma, cholangiocarcinoma, papillary thyroid cancer, secretory carcinoma of breast, breast cancer-unknown pathological type, and the like. The present inventors have conducted researches on compound 15 in the above patent in order to give a pharmaceutically acceptable crystal form.

### SUMMARY

The present disclosure is intended to provide a crystal form of a methanesulfonate of a compound of formula (I) and a preparation method therefor.

**The present disclosure provides a crystal form I of a methanesulfonate of a compound of formula (I):**
a crystal form I of a methanesulfonate of compound N-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 8.13±0.2°, 14.78±0.2°, 21.82±0.2°, 22.24±0.2°, 23.91±0.2°, 24.24±0.2°, 25.19±0.2°, and 26.94±0.2°,

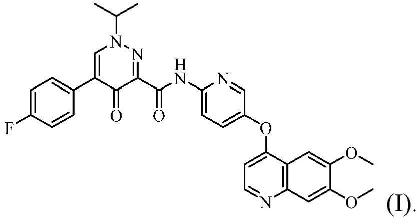

In one embodiment of the present disclosure, the crystal form I of the methanesulfonate of the compound of formula (I) further comprises characteristic peaks in an X-ray powder diffraction pattern using Cu-Kα radiation at 2θ angles of 11.11±0.2°, 17.37±0.2°, and 20.27±0.2°, in addition to the characteristic peaks described above.

In one embodiment of the present disclosure, the crystal form I of the methanesulfonate of the compound of formula (I) further comprises characteristic peaks in an X-ray powder diffraction pattern using Cu-Kα radiation at 2θ angles of 12.52±0.2°, 13.75±0.2°, and 19.13±0.2°, in addition to the characteristic peaks described above.

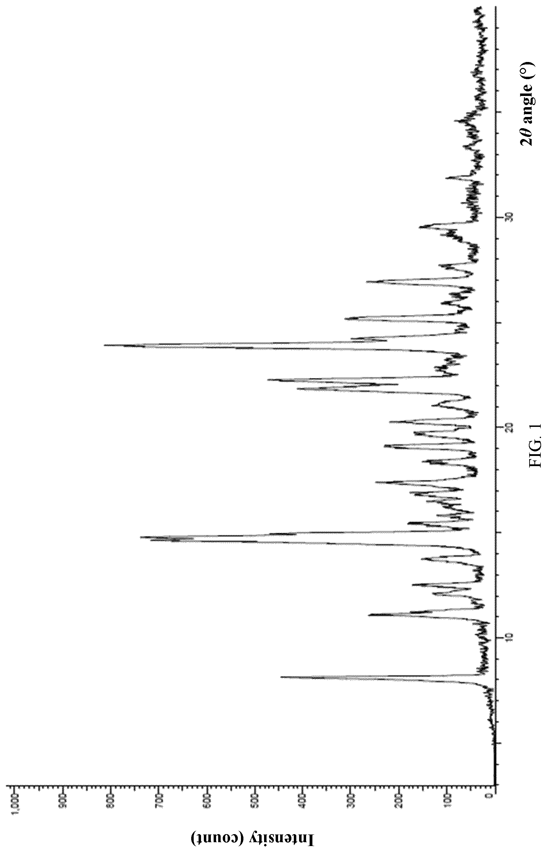

In one embodiment of the present disclosure, the crystal form I of the methanesulfonate of the compound of formula (I) has an X-ray powder diffraction pattern using Cu-Kα radiation as substantially shown in FIG. 1.

**The present disclosure further provides a preparation method for the crystal form I of the methanesulfonate of the compound of formula (I), which comprises:**
dissolving the compound of formula (I) in a single or mixed solvent, stirring until complete dissolution, then slowly adding methanesulfonic acid while stirring, and stirring until the crystal form I is precipitated.

In one embodiment of the present disclosure, the single or mixed solvent is selected from one of or a mixture of two or more of isopropanol, methanol, ethanol, tetrahydrofuran, dichloromethane, 1,4-dioxane, toluene, acetone, ethyl acetate, acetonitrile, methyl tert-butyl ether, 2-methyltetrahydrofuran, dimethyl sulfoxide, and water.

In one embodiment of the present disclosure, the single or mixed solvent is selected from methanol, ethanol, tetrahydrofuran, dichloromethane, 1,4-dioxane, and a mixed solution of isopropanol/ethanol.

In one embodiment of the present disclosure, a ratio of the mixed solution of isopropanol/ethanol is selected from 1:1 to 6:1, preferably selected from 2:1 to 5:1, and more preferably selected from 2:1 to 3:1.

In one embodiment of the present disclosure, when the single or mixed solvent is selected from isopropanol and 1,4-dioxane, the stirring is performed under a heating condition, wherein a temperature of the heating refers to 40 °C-60 °C, preferably 45 °C-55 °C.

In one embodiment of the present disclosure, the stirring is performed at room temperature or under a heating condition, wherein the room temperature or the heating condition refers to 0 °C-70 °C, preferably 10 °C-60 °C. Preferably, the room temperature refers to 10 °C-30 °C, preferably 15 °C-25 °C; preferably, the heating refers to 40 °C-60 °C, further preferably 45 °C-55 °C.

In one embodiment of the present disclosure, the room temperature refers to 15 °C-25 °C.

In one embodiment of the present disclosure, the heating refers to heating to above 30 °C.

In one embodiment of the present disclosure, the single or mixed solvent is used in an amount of 20-30 times the volume of the compound of formula (I).

In one embodiment of the present disclosure, the single or mixed solvent is used in an amount of 5-50 times, preferably 6-40 times, further preferably 7-35 times, and more preferably 8-25 times the volume of the compound of formula (I).

**The present disclosure provides a crystal form II of a methanesulfonate of a compound of formula (I):**

a crystal form II of a methanesulfonate of compound N-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 12.66±0.2°, 13.68±0.2°, 15.74±0.2°, 18.57±0.2°, 21.16±0.2°, and 22.19±0.2°,

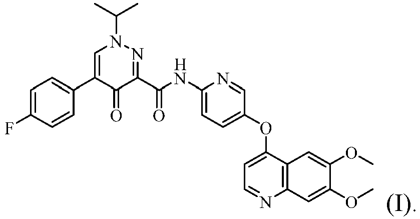

In one embodiment of the present disclosure, the crystal form II of the methanesulfonate of the compound of formula (I) further comprises characteristic peaks in an X-ray powder diffraction pattern using Cu-Kα radiation at 2θ angles of 18.09±0.2°, 20.13±0.2°, 24.88±0.2°, and 26.31±0.2°, in addition to the characteristic peaks described above.

In one embodiment of the present disclosure, the crystal form II of the methanesulfonate of the compound of formula (I) further comprises characteristic peaks in an X-ray powder diffraction pattern using Cu-Kα radiation at 2θ angles of 10.02±0.2°, 16.36±0.2°, and 29.41±0.2°, in addition to the characteristic peaks described above.

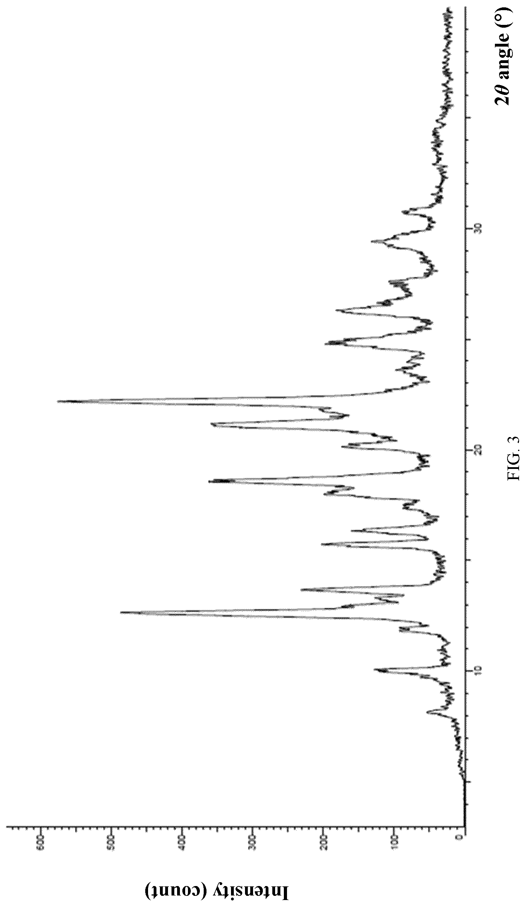

In one embodiment of the present disclosure, the crystal form II of the methanesulfonate of the compound of formula (I) has an X-ray powder diffraction pattern using Cu-Kα radiation as substantially shown in FIG. 3.

The present disclosure further provides a preparation method for the crystal form II of the methanesulfonate of the compound of formula (I), which comprises:
dissolving the compound of formula (I) in isopropanol, stirring at room temperature until complete dissolution, then slowly adding methanesulfonic acid while stirring, and stirring at room temperature until the crystal form II is precipitated.

In one embodiment of the present disclosure, the room temperature refers to 10 °C-30 °C, preferably 15 °C-25 °C.

**The present disclosure provides a crystal form III of a methanesulfonate of a compound of formula (I):**
a crystal form III of a methanesulfonate of compound *N*-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 10.32±0.2°, 10.58±0.2°, 12.55±0.2°, 17.30±0.2°, 21.49±0.2°, 21.95±0.2°, and 24.22±0.2°,

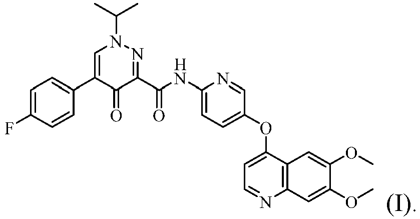

In one embodiment of the present disclosure, the crystal form III of the methanesulfonate of the compound of formula (I) further comprises characteristic peaks in an X-ray powder diffraction pattern using Cu-Kα radiation at 2θ angles of 9.47±0.2°, 13.04±0.2°, 20.66±0.2°, and 23.61±0.2°, in addition to the characteristic peaks described above.

In one embodiment of the present disclosure, the crystal form III of the methanesulfonate of the compound of formula (I) further comprises characteristic peaks in an X-ray powder diffraction pattern using Cu-Kα radiation at 2θ angles of 8.61±0.2°, 15.34±0.2°, and 25.71±0.2°, in addition to the characteristic peaks described above.

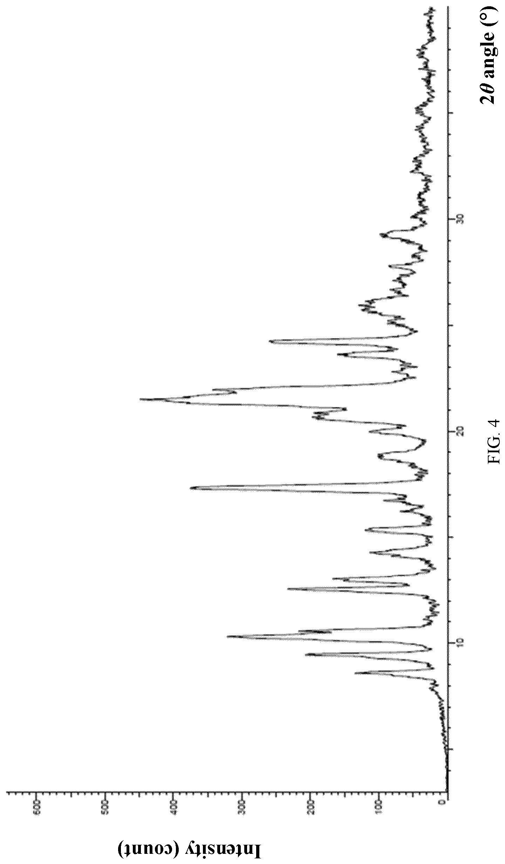

In one embodiment of the present disclosure, the crystal form III of the methanesulfonate of the compound of formula (I) has an X-ray powder diffraction pattern using Cu-Kα radiation as substantially shown in FIG. 4.

The present disclosure further provides a preparation method for the crystal form III of the methanesulfonate of the compound of formula (I), which comprises: dissolving the methane sulfonate of the compound of formula (I) in acetonitrile, slurrying, and precipitating the crystal form III.

The present disclosure further provides a preparation method for the crystal form III of the methanesulfonate of the compound of formula (I), which comprises: dissolving the crystal form I of the methanesulfonate of the compound of formula (I) in acetonitrile, slurrying, and precipitating the crystal form III.

In one embodiment of the present disclosure, the slurrying is performed under a condition of a temperature selected from 0 °C-70 °C, preferably selected from 10 °C-60 °C.

**The present disclosure provides a crystal form IV of a methanesulfonate of a compound of formula (I):**
a crystal form IV of a methanesulfonate of compound *N*-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 9.71±0.2°, 12.66±0.2°, 17.59±0.2°, 19.35±0.2°, 22.01±0.2°, 23.40±0.2°, and 24.77±0.2°,

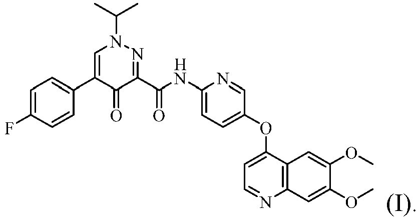

In one embodiment of the present disclosure, the crystal form IV of the methanesulfonate of the compound of formula (I) further comprises characteristic peaks in an X-ray powder diffraction pattern using Cu-Kα radiation at 2θ angles of 15.11±0.2°, 22.34±0.2°, and 23.92±0.2°, in addition to the characteristic peaks described above.

In one embodiment of the present disclosure, the crystal form IV of the methanesulfonate of the compound of formula (I) further comprises characteristic peaks in an X-ray powder diffraction pattern using Cu-Kα radiation at 2θ angles of 10.26±0.2°, 11.78±0.2°, and 12.45±0.2°, in addition to the characteristic peaks described above.

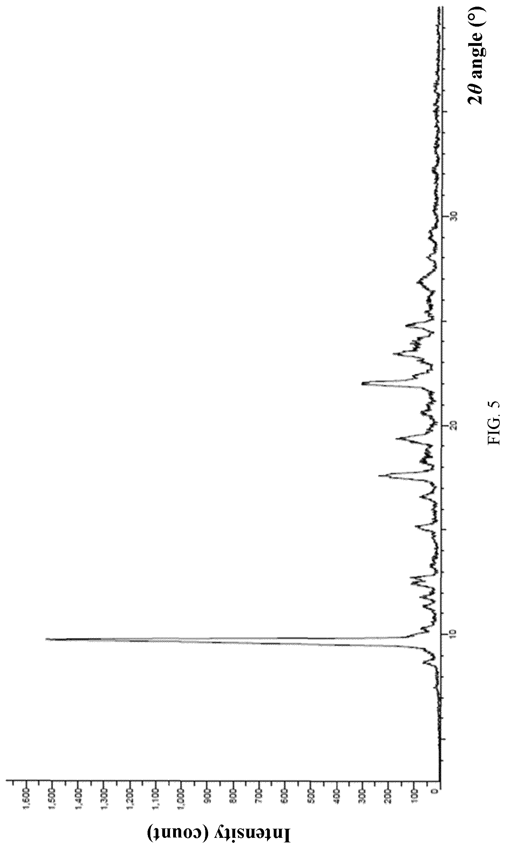

In one embodiment of the present disclosure, the crystal form IV of the methanesulfonate of the compound of formula (I) has an X-ray powder diffraction pattern using Cu-Kα radiation as substantially shown in FIG. 5.

The present disclosure further provides a preparation method for the crystal form IV of the methanesulfonate of the compound of formula (I), which comprises: dissolving the methanesulfonate of the compound of formula (I) in dichloromethane, slurrying, and precipitating the crystal form IV.

The present disclosure further provides a preparation method for the crystal form IV of the methanesulfonate of the compound of formula (I), which comprises: dissolving the crystal form I of the methanesulfonate of the compound of formula (I) in dichloromethane, slurrying, and precipitating the crystal form IV.

In one embodiment of the present disclosure, the slurrying is performed under a condition of a temperature selected from 0 °C-70 °C, preferably selected from 10 °C-60 °C, further preferably selected from 10 °C-30 °C, and more preferably selected from 15 °C-25 °C.

The present disclosure further provides a pharmaceutical composition comprising the crystal form I, II, III, or IV of the methanesulfonate of the compound of formula (I), and one or more second therapeutically active agents.

The present disclosure further provides a pharmaceutical formulation comprising the crystal form I, II, III, or IV of the methanesulfonate of the compound of formula (I).

In some embodiments of the present disclosure, the pharmaceutical formulation may comprise one or more pharmaceutical carriers.

The pharmaceutical carrier described herein may be one or more solid or liquid fillers suitable for administration in humans. The pharmaceutical carrier preferably has sufficient purity and sufficiently low toxicity, and is compatible with the compound provided herein without significantly decreasing its efficacy. For example, the pharmaceutical carrier may be a filler, a binder, a disintegrant, a lubricant, an aqueous solvent, a non-aqueous solvent, or the like.

The pharmaceutical formulation described herein may be formulated into any pharmaceutically acceptable dosage form to administer a "therapeutically effective amount" of the aforementioned crystal form I, II, III, or IV of the methanesulfonate of the compound of formula (I) to a patient or a subject in need of such treatment in any suitable route of administration, such as oral, parenteral, rectal, or pulmonary administration. For oral administration, it may be formulated into tablets, capsules, pills, granules, and the like. For parenteral administration, it may be formulated into solution injections, sterile powders for injection, and the like.

The present disclosure further provides use of the crystal form I, II, III, or IV of the methanesulfonate of the compound of formula (I), or the pharmaceutical formulation or the pharmaceutical composition comprising the crystal form I, II, III, or IV in the manufacture of a medicament for treating and/or preventing a related disease caused by an abnormal signaling pathway resulting from abnormal expression of a TAM family kinase receptor and/or CSF 1R kinase receptor and/or a ligand thereof.

In some embodiments of the present disclosure, the disease mediated by the abnormal expression of the TAM family kinase receptor and/or CSF 1R kinase receptor and/or the ligand thereof includes at least one of the following diseases: tumor, immune dysregulation, endometriosis, vascular disease/trauma, psoriasis, visual defect/lesion (caused by macular degeneration, diabetes, premature delivery, etc.), kidney disease, rheumatoid arthritis, osteoporosis, and other related diseases.

The present disclosure further provides use of the crystal form I, II, III, or IV of the methanesulfonate of the compound of formula (I), or the pharmaceutical formulation or the pharmaceutical composition comprising the crystal form I, II, III, or IV in the manufacture of a medicament for treating and/or preventing an NTRK-caused related disease.

The present disclosure further provides use of the crystal form I, II, III, or IV of the methanesulfonate of the compound of formula (I), or the pharmaceutical formulation or the pharmaceutical composition comprising the crystal form I, II, III, or IV in the manufacture of a medicament for treating and/or preventing an NTRK-caused related disease, especially a related disease of drug-resistant type resulting from mutation of NTRK, wherein the NTRK-caused related disease includes, but is not limited to, non-small cell lung cancer, colorectal cancer, mammary analog secretory carcinoma, sarcoma, astrocytoma, glioblastoma, Spitz-like melanoma, cholangiocarcinoma, papillary thyroid cancer, secretory carcinoma of breast, breast cancer-unknown pathological type, and the like.

### Detailed Description of the Invention

The "room temperature" described herein refers to an indoor temperature, usually 10 °C-30 °C, and more usually 15 °C-25 °C.

The "times the volume" described herein refers to the volume (mL) of a solvent required to dissolve 1 g of a substance; for example, if 20 mL of a solvent is required to dissolve 1 g of a compound of formula (I), it is referred to as 20 times the volume.

The "ethanol" described herein includes absolute ethanol and a mixed solution of ethanol and water, and ethanol with a content of ≥ 95.0% is used in the present disclosure.

The "methanol" described herein is methanol with a content of ≥ 98%, including but not limited to analytical grade, industrial grade, and absolute methanol.

The "therapeutically effective amount" described herein refers to an amount of the aforementioned crystal form of the compound, the composition, or the pharmaceutical formulation that, when administered to a patient, is at least capable of alleviating symptoms of the patient's condition. An actual amount comprising the "therapeutically effective amount" will vary depending on a variety of circumstances, including, but not limited to, the particular condition being treated, the severity of the condition, the physique and health status of the patient, and the route of administration. The appropriate amount can be readily determined by skilled medical practitioners using methods known in the medical field.

### Beneficial Effects of the Present Disclosure

It is found by the research of the present disclosure that the crystal forms of the present disclosure have very good physicochemical properties and pharmaceutical stability, and also have a greater improvement in pharmacodynamic and pharmacokinetic properties, which are more conducive to drug development.

### BRIEF DESCRIPTION OF THE DRAWINGS

FIG. 1 is an X-ray powder diffraction (XRPD) pattern of the crystal form I of the compound of formula (I).
FIG. 2 is a differential scanning calorimetry (DSC) pattern of the crystal form I of the compound of formula (I).
FIG. 3 is an X-ray powder diffraction (XRPD) pattern of the crystal form II of the compound of formula (I).
FIG. 4 is an X-ray powder diffraction (XRPD) pattern of the crystal form III of the compound of formula (I).
FIG. 5 is an X-ray powder diffraction (XRPD) pattern of the crystal form IV of the compound of formula (I).

### DETAILED DESCRIPTION

The above description of the present disclosure is further illustrated in detail below by way of specific embodiments, but it should not be construed that the scope of the above subject matter of the present disclosure is limited to the following examples. All techniques implemented based on the above description of the present disclosure fall within the scope of the present disclosure.

### Abbreviations used herein:

### -Boc: tert-butyloxycarbonyl

"RH": relative humidity
"DMF": N,N-dimethylformamide
"DCM": dichloromethane
"DMSO": dimethyl sulfoxide
"FaSSIF": fasted state simulated intestinal fluid
"FeSSIF": fed state simulated intestinal fluid

### Preparation Example 1: Synthesis of intermediate 6-(4-fluorophenyl)-2-isopropyl-5-oxo-2,5-dihydropyridazine-4-carboxylic acid

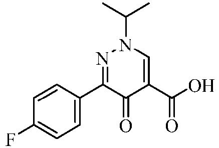

### Step 1: synthesis of tert-butyl 2-isopropylhydrazine-1-carboxylate

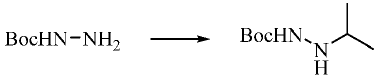

tert-Butyl hydrazinecarboxylate (20.0 g, 0.151 mol), acetone (9.26 g, 0.16 mol), acetic acid (2 mL), magnesium sulfate (10.0 g), and sodium triacetoxyborohydride (96.64 g, 0.456 mol) were added to dichloromethane (100 mL). The mixture was reacted at room temperature overnight. After the reaction was completed, as detected by TLC, the mixture was filtered, and the filtrate was concentrated under reduced pressure to give the product (25 g, yield: 95.6%).

### Step 2: synthesis of tert-butyl 2-(3-ethoxy-3-oxopropyl)-2-isopropylhydrazine-1-carboxylate

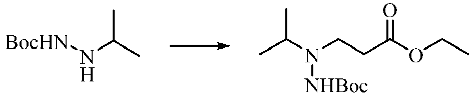

*tert*-Butyl 2-isopropylhydrazine-1-carboxylate (19.0 g, 0.109 mmol) and ethyl acrylate (12.01 g, 0.12 mol) were added to ethanol (60 mL), and the mixture was heated to 80 °C and reacted overnight. After the reaction was completed, as detected by TLC, the mixture was concentrated under reduced pressure, and water was added. The resulting mixture was extracted with ethyl acetate, and the organic phases were combined and dried over anhydrous sodium sulfate. The crude product was purified by silica gel column chromatography (DCM:MeOH = 200:1 to 80:1) to give the product (24.2 g, yield: 81.0%).

### Step 3: synthesis of ethyl 3-(1-isopropylhydrazineyl)propanoate

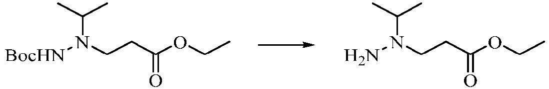

*tert*-Butyl 2-(3-ethoxy-3-oxopropyl)-2-isopropylhydrazine-1-carboxylate (24.1 g, 0.088 mol) was added to dichloromethane (75 mL), and the mixture was cooled to 0 °C under an ice bath. Trifluoroacetic acid (35 mL) was added, and the mixture was slowly warmed to room temperature and reacted overnight. After the reaction was completed, as detected by TLC, the mixture was concentrated under reduced pressure to give the product (18.9 g, crude product).

### Step 4: synthesis of ethyl 2-(4-fluorophenyl)-2-oxoacetate

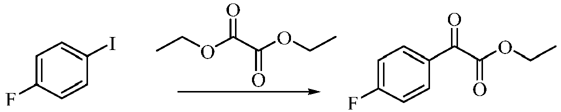

1-Fluoro-4-iodobenzene (10.0 g, 0.0451 mol) was added to 2-methyltetrahydrofuran (10 mL), and the mixture was cooled to 0 °C under an ice bath. Isopropylmagnesium chloride (2 mol/L, 24.78 mL, 0.049 mol) was added, and the mixture was stirred at 0 °C for 30 min. The above reaction solution was added dropwise to a solution of diethyl oxalate (7.25 g, 0.0496 mol) in 2-methyltetrahydrofuran (50 mL), and the mixture was slowly warmed to room temperature and reacted overnight. After the reaction was completed, as detected by TLC, the reaction solution was added to an aqueous citric acid solution, with the pH value maintained at 5-6. The aqueous phase was extracted with ethyl acetate, and the organic phases were washed with water, combined, washed with brine, dried over anhydrous sodium sulfate, and filtered. The filtrate was concentrated under reduced pressure, and the crude product was purified by silica gel column chromatography (PE:EA = 120:1 to 40:1) to give the product (8 g, yield: 90.5%).

### Step 5: synthesis of ethyl 3-(2-(2-ethoxy-1-(4-fluorophenyl)-2-oxoethylidene)-1-isopropylhydrazineyl)propanoate

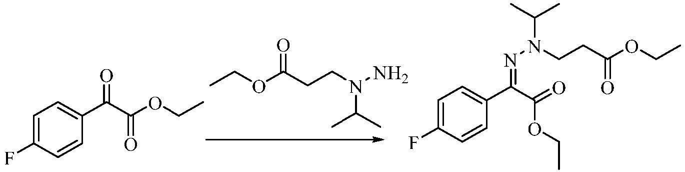

Ethyl 2-(4-fluorophenyl)-2-oxoacetate (4.43 g, 22.6 mmol) and ethyl 3-(1-isopropylhydrazineyl)propanoate (11.81 g, crude product) were added to ethanol (10 mL), and the mixture was heated to 80 °C and reacted overnight. After the reaction was completed, as detected by TLC, the mixture was filtered under vacuum, and water was added to the filtrate. The resulting mixture was extracted with ethyl acetate, and the organic phases were combined, dried over anhydrous sodium sulfate, and filtered. The filtrate was concentrated under reduced pressure, and the crude product was purified by silica gel column chromatography (PE:EA = 60:1 to 10:1) to give the product (2.3 g, yield: 28.9%).

### Step 6: synthesis of ethyl 6-(4-fluorophenyl)-2-isopropyl-5-oxo-2,3,4,5-tetrahydropyridazine-4-carboxylate

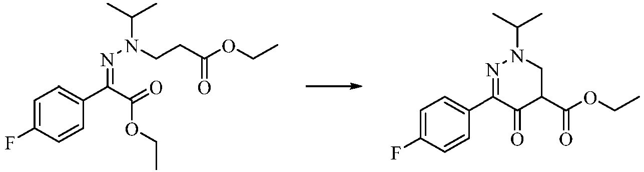

Ethyl 3-(2-(2-ethoxy-1-(4-fluorophenyl)-2-oxoethylidene)-1-isopropylhydrazineyl) propanoate (2.27 g, 6.45 mmol) was added to 2-methyltetrahydrofuran (10 mL), and the mixture was cooled to 0 °C under an ice bath. Potassium tert-butoxide (1.81 g, 16.11 mmol) was added, and the mixture was slowly warmed to room temperature and reacted overnight. After the reaction was completed, as detected by TLC, the reaction solution was added to an aqueous citric acid solution, with the pH value maintained at 5-6. The mixture was extracted with ethyl acetate, and the organic phases were combined, dried over anhydrous sodium sulfate, and filtered. The filtrate was concentrated under reduced pressure to give the product (1.27 g, yield: 64.5%).

### Step 7: synthesis of ethyl 6-(4-fluorophenyl)-2-isopropyl-5-oxo-2,5-dihydropyridazine-4-carboxylate

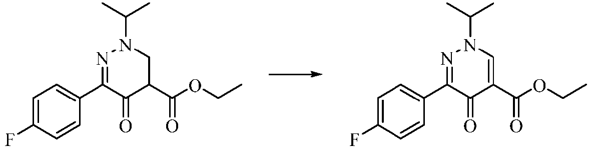

Ethyl 6-(4-fluorophenyl)-2-isopropyl-5-oxo-2,3,4,5-tetrahydropyridazine-4-carboxylate (1.2 g, 3.92 mmol) and copper(II) chloride dihydrate (2.67 g, 15.68 mmol) were added to acetonitrile (10 mL), and the mixture was reacted at 80 °C for 4 h. After the reaction was completed, as detected by TLC, water was added to the reaction solution, and the mixture was extracted with ethyl acetate. The organic phases were combined, washed with water, washed with saturated brine, dried over anhydrous sodium sulfate, and filtered. The filtrate was concentrated under reduced pressure, and the crude product was purified by silica gel column chromatography (PE:EA = 100:1 to 40:1) to give the product (730.0 mg, yield: 61.2%).

### Step 8: synthesis of 6-(4-fluorophenyl)-2-isopropyl-5-oxo-2,5-dihydropyridazine-4-carboxylic acid

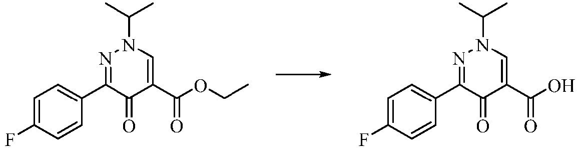

Ethyl 6-(4-fluorophenyl)-2-isopropyl-5-oxo-2,5-dihydropyridazine-4-carboxylate (700.0 mg, 2.3 mmol) was added to a mixed solvent of methanol (6 mL) and water (5 mL), and lithium hydroxide monohydrate (284.4 mg, 6.91 mmol) was added. The mixture was stirred at room temperature for 1 h. After the reaction was completed, as detected by TLC, the pH value was adjusted to 2-3 with citric acid, and a solid precipitated. The mixture was filtered under vacuum, and the filter cake was dissolved in dichloromethane, dried over anhydrous sodium sulfate, and filtered. The filtrate was concentrated under reduced pressure to give the product (600.0 mg, yield: 94.5%).

### Example 1: Preparation of compound N-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I)

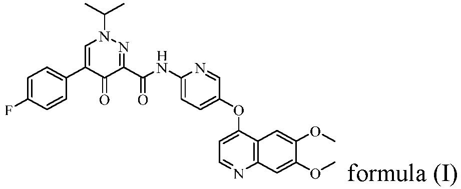

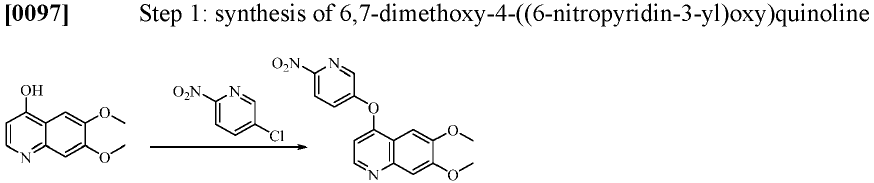

6,7-Dimethoxyquinolin-4-ol (15.00 g, 73.10 mmol, 1.0 eq), 5-chloro-2-nitropyridine (11.60 g, 73.10 mmol, 1.0 eq), and K₂CO₃ (20.20 g, 146.11 mmol, 2.0 eq) were added to DMF (120 mL), and the mixture was stirred at 80 °C overnight in nitrogen atmosphere. After the reaction was completed, as detected by TLC, the mixture was filtered. The filter cake was rinsed with dichloromethane, and the filtrate was concentrated under reduced pressure. The concentrate was dissolved in dichloromethane (50 mL), washed successively with distilled water (20 × 4 mL) and saturated brine (20 mL), dried over anhydrous sodium sulfate, and filtered under vacuum. The filtrate was concentrated under reduced pressure, and the crude product was purified by silica gel column chromatography (PE:EA = 1:9 to EA) to give the product (4.70 g, yield: 19.6%).

### Step 2: synthesis of 5-((6,7-dimethoxyquinolin-4-yl)oxy)pyridin-2-amine

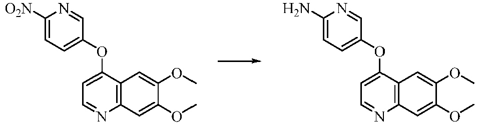

6,7-Dimethoxy-4-((6-nitropyridin-3-yl)oxy)quinoline (4.70 g, 14.36 mmol, 1.0 eq), reduced iron powder (4.81 g, 86.16 mmol, 6.0 eq), and NH₄Cl (9.22 g, 172.32 mmol, 12.0 eq) were added to a mixed solvent of ethanol (100 mL) and water (40 mL), and the mixture was stirred at 80 °C for 4 h. After the reaction was completed, as detected by TLC, the mixture was filtered while hot. The filter cake was rinsed with dichloromethane, and the filtrate was concentrated under reduced pressure. The aqueous phase was extracted with dichloromethane (20 mL × 3), and the organic phases were combined, washed with saturated brine (20 mL), dried over anhydrous sodium sulfate, and filtered under vacuum. The filtrate was concentrated under reduced pressure to give the product (4.07 g, yield: 95.3%).

### Step 3: synthesis of N-(5-((6,7-dimethoxyquinolin-4-yl)oxy)pyridin-2-yl)-5-(4-fluorophenyl)-1-isopropyl-4-oxo-1,4-dihydropyridazine-3-carboxamide

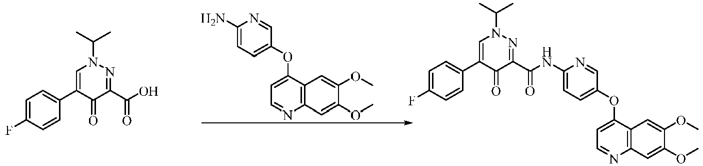

1-Isopropyl-4-oxo-5-(4-fluorophenyl)-1,4-dihydropyridazine-3-carboxylic acid (149.0 mg, 0.54 mmol, 1.0 eq) and 5-((6,7-dimethoxyquinolin-4-yl)oxy)pyridin-2-amine (192.4 mg, 0.65 mmol, 1.2 eq) were dissolved in anhydrous pyridine (2 mL), and the mixture was stirred for 10 min. POCl₃ was slowly added dropwise until the solid was dissolved. After the reaction was completed, as detected by TLC, the mixture was concentrated under reduced pressure. The concentrate was dissolved in ethyl acetate (10 mL), washed successively with 1 mol/L hydrochloric acid (5 mL), a saturated aqueous sodium bicarbonate solution (5 mL), water (5 mL × 2), and saturated brine (5 mL), dried over anhydrous sodium sulfate, and filtered under vacuum. The filtrate was concentrated under reduced pressure, and the crude product was purified by silica gel column chromatography (PE:EA = 1:1) to give the product (151 mg, yield: 50.4%).

¹H NMR (400MHz, DMSO-*d*₆) δ(ppm): 13.08 (s, 1H), 8.94 (s, 1H), 8.52-8.50 (d, 1H), 8.44-8.42 (t, 2H), 7.98-7.95 (m, 2H), 7.91-7.88 (q, 1H), 7.55 (s, 1H), 7.42 (s, 1H), 7.37-7.33 (t, 2H), 6.60-6.58 (d, 1H), 4.80-4.74 (m, 1H), 3.96-3.95 (d, 6H), 1.54-1.53 (d, 6H).

Molecular formula: C₃₀H₂₆FN₅O₅ Molecular weight: 555.57 LC-MS (Pos, *m*/*z*) = 556.33[M+H]⁺.

### Example 2: Preparation of crystal form I of methanesulfonate of compound of formula (I)

0.56 g of the compound of formula (I) was weighed and placed into a reaction flask, and 5 mL of absolute methanol was added. The mixture was stirred at room temperature for 0.5 h, and then 78 µL of methanesulfonic acid was slowly added while stirring. The mixture was stirred at room temperature for crystallization for 20 h and filtered, and the filter cake was dried under vacuum at 50 °C to give the crystal form I of the methanesulfonate.

The crystal form I comprises characteristic peaks in an X-ray powder diffraction pattern using Cu-Kα radiation at 2*θ* (°) angles of 8.13±0.2°, 14.78±0.2°, 21.82±0.2°, 22.24±0.2°, 23.91±0.2°, 24.24±0.2°, 25.19±0.2°, and 26.94±0.2°; the crystal form I further comprises characteristic peaks at 2*θ* (°) angles of 11.11±0.2°, 17.37±0.2°, and 20.27±0.2°; the crystal form I further comprises characteristic peaks at 2*θ* (°) angles of 12.52±0.2°, 13.75±0.2°, and 19.13±0.2°. The XRPD analysis is as shown in FIG. 1.

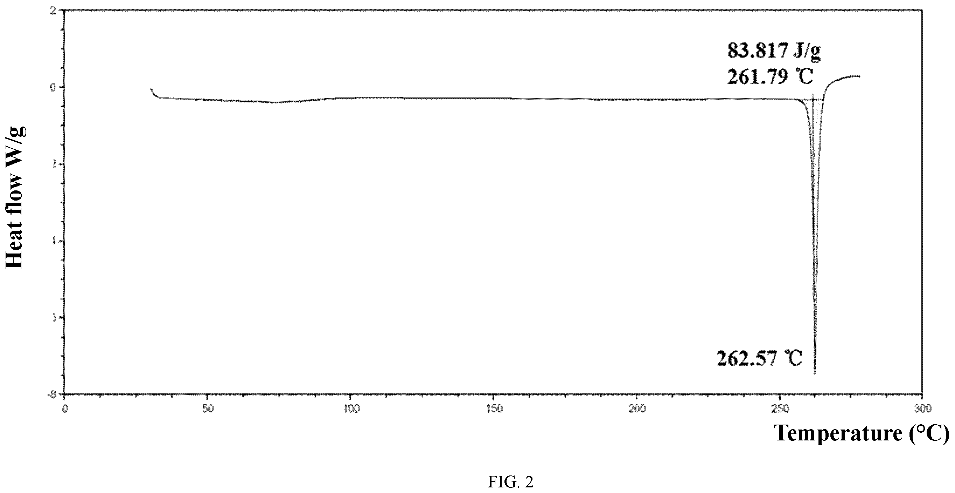

As measured by a differential scanning calorimeter, the melting temperature of the crystal form I was about 261 °C-263 °C, as shown in FIG. 2.

### Example 3: Preparation of crystal form I of methanesulfonate of compound of formula (I)

0.56 g of the compound of formula (I) was weighed and placed into a reaction flask, and 5 mL of absolute ethanol was added. The mixture was stirred at room temperature for 0.5 h, and then 78 µL of methanesulfonic acid was slowly added while stirring. The mixture was stirred at room temperature for crystallization for 20 h and filtered, and the filter cake was dried under vacuum at 50 °C to give the crystal form I of the methanesulfonate.

An X-ray powder diffraction pattern using Cu-Kα radiation of the crystal form I is as substantially shown in FIG. 1.

### Example 4: Preparation of crystal form I of methanesulfonate of compound of formula (I)

0.56 g of the compound of formula (I) was weighed and placed into a reaction flask, and 5 mL of tetrahydrofuran was added. The mixture was stirred at room temperature for 10 min, and then 78 µL of methanesulfonic acid was slowly added while stirring. The mixture was stirred at room temperature for crystallization for 20 h and filtered, and the filter cake was dried under vacuum at 50 °C to give the crystal form I of the methanesulfonate.

An X-ray powder diffraction pattern using Cu-Kα radiation of the crystal form I is as substantially shown in FIG. 1.

### Example 5: Preparation of crystal form I of methanesulfonate of compound of formula (I)

0.56 g of the compound of formula (I) was weighed and placed into a reaction flask, and 5 mL of dichloromethane was added. The mixture was stirred at room temperature for 10 min, and then 78 µL of methanesulfonic acid was slowly added while stirring. The mixture was stirred at room temperature for crystallization for 20 h and filtered, and the filter cake was dried under vacuum at 50 °C to give the crystal form I of the methanesulfonate.

An X-ray powder diffraction pattern using Cu-Kα radiation of the crystal form I is as substantially shown in FIG. 1.

### Example 6: Preparation of crystal form I of methanesulfonate of compound of formula (I)

0.56 g of the compound of formula (I) was weighed and placed into a reaction flask, and 7 mL of a mixed solution of isopropanol/ethanol (isopropanol:ethanol = 5:2) was added. The mixture was stirred at room temperature for 20 min, and then 78 µL of methanesulfonic acid was slowly added while stirring. The mixture was stirred at room temperature for crystallization for 20 h and filtered, and the filter cake was dried under vacuum at 50 °C to give the crystal form I of the methanesulfonate.

An X-ray powder diffraction pattern using Cu-Kα radiation of the crystal form I is as substantially shown in FIG. 1.

### Example 7: Preparation of crystal form I of methanesulfonate of compound of formula (I)

0.56 g of the compound of formula (I) was weighed and placed into a reaction flask, and 6 mL of isopropanol was added. The mixture was stirred at 50 °C for 0.5 h, and then 78 µL of methanesulfonic acid was slowly added while stirring. The mixture was stirred at 50 °C for 2 h, and then the heating was stopped. The mixture was naturally cooled to room temperature while stirring, stirred for another 16 h, and then filtered, and the filter cake was dried under vacuum at 50 °C to give the crystal form I of the methanesulfonate.

An X-ray powder diffraction pattern using Cu-Kα radiation of the crystal form I is as substantially shown in FIG. 1.

### Example 8: Preparation of crystal form I of methanesulfonate of compound of formula (I)

0.56 g of the compound of formula (I) was weighed and placed into a reaction flask, and 10 mL of 1,4-dioxane was added. The mixture was stirred at 50 °C for 0.5 h, and then 78 µL of methanesulfonic acid was slowly added while stirring. The mixture was stirred at 50 °C for 2 h, and then the heating was stopped. The mixture was naturally cooled to room temperature while stirring, stirred for another 16 h, and then filtered, and the filter cake was dried under vacuum at 50 °C to give the crystal form I of the methanesulfonate. An X-ray powder diffraction pattern using Cu-Kα radiation of the crystal form I is as substantially shown in FIG. 1.

### Example 9: Preparation of crystal form II of methanesulfonate of compound of formula (I)

1.12 g of the compound of formula (I) was weighed and placed into a reaction flask, and 5 mL of isopropanol was added. The mixture was stirred at room temperature for 0.5 h, and then 156 µL of methanesulfonic acid was slowly added while stirring. The mixture was stirred at room temperature for crystallization for 20 h and filtered, and the filter cake was dried under vacuum at 50 °C to give the crystal form II of the methanesulfonate.

The crystal form II comprises characteristic peaks in an X-ray powder diffraction pattern using Cu-Kα radiation at 2*θ* (°) angles of 12.66±0.2°, 13.68±0.2°, 15.74±0.2°, 18.57±0.2°, 21.16±0.2°, and 22.19±0.2°; the crystal form II further comprises characteristic peaks at 2*θ* (°) angles of 18.09±0.2°, 20.13±0.2°, 24.88±0.2°, and 26.31±0.2°; the crystal form II further comprises characteristic peaks at 2*θ* (°) angles of 10.02±0.2°, 16.36±0.2°, and 29.41±0.2°. The XRPD analysis is as shown in FIG. 3.

### Example 10: Preparation of crystal form III of methanesulfonate of compound of formula (I)

1 g of the crystal form I of the methanesulfonate was weighed and placed into a reaction flask, and 10 mL of acetonitrile was added. The mixture was slurried at room temperature or 50 °C for 24 h and filtered, and the filter cake was dried under vacuum at 50 °C to give the crystal form III of the methanesulfonate.

The crystal form III comprises characteristic peaks in an X-ray powder diffraction pattern using Cu-Kα radiation at 2*θ* (°) angles of 10.32±0.2°, 10.58±0.2°, 12.55±0.2°, 17.30±0.2°, 21.49±0.2°, 21.95±0.2°, and 24.22±0.2°; the crystal form III further comprises characteristic peaks at 2*θ* (°) angles of 9.47±0.2°, 13.04±0.2°, 20.66±0.2°, and 23.61±0.2°; the crystal form III further comprises characteristic peaks at 2*θ* (°) angles of 8.61±0.2°, 15.34±0.2°, and 25.71±0.2°. The XRPD analysis is as shown in FIG. 4.

### Example 11: Preparation of crystal form IV of methanesulfonate of compound of formula (I)

1 g of the crystal form I of the methanesulfonate was weighed and placed into a reaction flask, and 30 mL of dichloromethane was added. The mixture was slurried at room temperature for 24 h and filtered, and the filter cake was dried under vacuum at 50 °C to give the crystal form IV of the methanesulfonate.

The crystal form IV comprises characteristic peaks in an X-ray powder diffraction pattern using Cu-Kα radiation at 2*θ* (°) angles of 9.71±0.2°, 12.66±0.2°, 17.59±0.2°, 19.35±0.2°, 22.01±0.2°, 23.40±0.2°, and 24.77±0.2°; the crystal form IV further comprises characteristic peaks at 2*θ* (°) angles of 15.11±0.2°, 22.34±0.2°, and 23.92±0.2°; the crystal form IV further comprises characteristic peaks at 2*θ* (°) angles of 10.26±0.2°, 11.78±0.2°, and 12.45±0.2°. The XRPD analysis is as shown in FIG. 5.

The present disclosure can be better understood according to the following experimental examples. However, it is easily understood by those skilled in the art that the contents described in the experimental examples are only used to illustrate the present disclosure, and should not and will not limit the present disclosure described in detail in the claims.

### Experimental Example 1: Assay of inhibitory activity of crystal form of the present disclosure on cell AXL

H1299 is a non-small cell lung cancer cell line.

Test substance: the crystal form of the **methanesulfonate** of the compound of formula (I) of the present disclosure, having the structure shown above.

Test instruments: protein electrophoresis apparatus (Bio Rad), membrane transfer apparatus (Bio Rad), exposure apparatus (Tanon), CO₂ cell incubator (Thermo), and the like.

### Assay method:

H1299 cells were inoculated into a 6-well plate (containing 10% FBS 1640 medium, 5 × 10⁵ cells/well) and adherently cultivated at 37 °C with 5% CO₂ for 18 h. The cells were starved overnight, and different concentrations of the compound were added. The final compound concentrations were 0.37 nM, 1.1 nM, 3.3 nM, 10 nM, and 30 nM, with the final DMSO content in each compound well being 1 ‰. The negative control well contained medium with 1‰ DMSO. The cells were incubated at 37 °C with 5% CO₂ for 60 min, and then hGAS6 (R&D, final concentration: 200 ng/mL) was added to each well. The cells were incubated for another 60 min, and then the total protein of the cells was extracted for a Western Blot experiment to assay the inhibitory activity of the compound on cell pAXL.

The assay results are shown in Table 1.

**Table 1. Inhibitory activity of crystal form of the present disclosure on cell pAXL**

| Test substance | IC₅₀ (nM) |
|---|---|
| Crystal form I | <0.37 |

It can be seen from the experimental results in Table 1 that the crystal form of the methanesulfonate of the compound of formula (I) of the present disclosure has a significant inhibitory effect on H1299 cell pAXL. It demonstrates that the crystal form of the methanesulfonate of the compound of formula (I) of the present disclosure can effectively inhibit the activity of AXL at the cellular level, thus being a prominent AXL inhibitor.

### Experimental Example 2: PK Evaluation of crystal forms of the present disclosure in rats

### Animal administration and sample collection:

The experimental crystal forms I and II of the methanesulfonate of the compound of formula (I) were each prepared into a suspension solution with 0.5% HPMC. The compound solutions were intragastrically administered to SD rats at a dose of 60 mg/kg. Blood samples were collected at time points of 15 min, 30 min, 1 h, 2 h, 4 h, 6 h, 8 h, 24 h, 48 h, 72 h, and 96 h after administration.

The animals were fixed, with tails warmed in a water bath 10 min before each sampling time point. About 100 µL of blood was collected via the tail vein, and the blood samples were collected in anticoagulation tubes containing EDTA-K₂. The blood samples were then centrifuged at 8,000 rpm for 6 min at 4 °C to give plasma samples. The plasma samples were prepared within 30 min after blood collection. The plasma samples were stored in a refrigerator at -80 °C before the test.

### Sample analysis method:

The sample to be tested was taken out from the -80 °C refrigerator, naturally thawed at room temperature, and vortexed for 5 min. 20 µL of plasma sample was precisely pipetted to a 1.5 mL centrifuge tube, and 200 µL of internal standard working solution (a solution of tolbutamide in methanol, 100 ng/mL) was added. The resulting mixture was thoroughly mixed, vortexed for 5 min, and centrifuged at 12,000 rpm for 5 min. 50 µL of supernatant was precisely pipetted to a 96-well plate pre-loaded with 150 µL of water per well, and the resulting mixture was vortexed for 5 min and then used for LC-MS/MS analysis.

### Data processing method:

The concentrations of the test samples were calculated by Analyst 1.6.3 from AB Sciex. Parameters such as mean, standard deviation, and coefficient of variation (excluding those output directly by Analyst 1.6.3) were calculated by Microsoft Excel. PK parameters were calculated by Pharsight Phoenix 6.1 software NCA (Tₘₐₓ was in median).

The results are shown in Table 2 below.

**Table 2. PK parameters of different crystal forms in SD rats (PO: 60 mg/kg, n = 3)**

| Test substance | λ_z (1/h) | t_{z1/2} (h) | Tₘₐₓ (h) | Cₘₐₓ (ng/mL) | AUCₗₐₛₜ (h*ng/mL) |
|---|---|---|---|---|---|
| Crystal form I | 0.018 | 38.6 | 8.00 | 68967 | 4016843 |
| Crystal form II | 0.026 | 27.0 | 6.00 | 58933 | 3054395 |

Notes: t_{z1/2}: terminal half-life; Tₘₐₓ: time to maximum plasma concentration; AUCₗₐₛₜ: area under plasma concentration-time curve, 0-96 h.

It can be seen from the experimental results in Table 2 that the crystal forms of the methanesulfonate of the compound of formula (I) of the present disclosure have good pharmacokinetic characteristics and good druggability.

### Experimental Example 3: Stability test of crystal forms of the present disclosure

Test substances: the crystal forms I, II, III, and IV of the methanesulfonate of the compound of formula (I) of the present disclosure.

Methodology: Appropriate amounts of samples of the crystal forms I, II, III, and IV of the methanesulfonate of the compound of formula (I) were separately left to stand open under the conditions of a high temperature (60 °C), a high humidity (RH 92.5%), and illumination (4500±500 Lux); sampling was performed on days 5, 10, and 30 to investigate changes in the appearance, purity, and crystal form of the samples.

### Results:

**Table 3. Test results under influencing factors**

| Conditions of standing | Appearance | | | | Purity (%) | | | |
|---|---|---|---|---|---|---|---|---|
| | Crystal form I | Crystal form II | Crystal form III | Crystal form IV | Crystal form I | Crystal form II | Crystal form III | Crystal form IV |
| 0 days | White or off-white solid | White or off-white solid | White or off-white solid | White or off-white solid | 99.69 | 99.74 | 99.90 | 99.91 |
| Left to stand open under illumination for 5 days | White or off-white solid | White or off-white solid | White or off-white solid | White or off-white solid | 99.70 | 99.75 | 99.90 | 99.91 |
| Left to stand open at 60 °C for 5 days | White or off-white solid | White or off-white solid | White or off-white solid | White or off-white solid | 99.70 | 99.74 | 99.90 | 99.91 |
| Left to stand open at RH 92.5% for 5 days | White or off-white solid | White or off-white solid | White or off-white solid | White or off-white solid | 99.69 | 99.74 | 99.90 | 99.92 |
| Left to stand open under illumination for 10 days | White or off-white solid | White or off-white solid | White or off-white solid | White or off-white solid | 99.69 | 99.74 | 99.91 | 99.91 |
| Left to stand open at 60 °C for 10 days | White or off-white solid | White or off-white solid | White or off-white solid | White or off-white solid | 99.68 | 99.73 | 99.90 | 99.91 |
| Left to stand open at RH 92.5% for 10 days | White or off-white solid | White or off-white solid | White or off-white solid | White or off-white solid | 99.68 | 99.74 | 99.90 | 99.92 |
| Left to stand open under illumination for 30 days | White or off-white solid | White or off-white solid | White or off-white solid | White or off-white solid | 99.69 | 99.75 | 99.91 | 99.88 |
| Left to stand open at 60 °C for 30 days | White or off-white solid | White or off-white solid | White or off-white solid | White or off-white solid | 99.67 | 99.72 | 99.89 | 99.87 |
| Left to stand open at RH 92.5% for 30 days | White or off-white solid | White or off-white solid | White or off-white solid | White or off-white solid | 99.67 | 99.74 | 99.90 | 99.92 |

Conclusion: After being left to stand under conditions of the influencing factors for 30 days, the crystal forms I, II, III, and IV of the methanesulfonate of the compound of formula (I) of the present disclosure showed no significant changes in the appearance and purity, indicating that the crystal forms had good stability.

### Experimental Example 4: Determination of solubility of crystal forms of the present disclosure in test solutions with different pH values

**Test substances:** the crystal forms I, II, III, and IV of the methanesulfonate of the compound of formula (I).

### Preparation of buffer solutions with different pH values:

### (1) FaSSIF (pH 6.5):

1) 0.42 g of NaOH, 3.44 g of anhydrous NaH₂PO₄, and 6.19 g of NaCl were dissolved in about 0.9 L of pure water;
2) the pH was adjusted to 6.5 using 1 N NaOH or 1 N HCl, and the buffer solution was supplemented to the volume of 1 L with pure water at room temperature;
3) 2.24 g of FaSSIF powder was added to about 0.5 L of the buffer solution, and the mixture was stirred until the powder was completely dissolved; the resulting solution was supplemented to the volume of 1 L with the buffer solution at room temperature, and after being left to stand for 2 h, it was ready for use.

### (2) FeSSIF (pH 5.0):

1) 4.04 g of NaOH, 8.65 g of glacial acetic acid, and 11.87 g of NaCl were dissolved in about 0.9 L of pure water;
2) the pH was adjusted to 5.0 using 1 N NaOH or 1 N HCl, and the buffer solution was supplemented to the volume of 1 L with pure water at room temperature;
3) 11.2 g of FeSSIF powder was added to about 0.5 L of the buffer solution, and the mixture was stirred until the powder was completely dissolved; the resulting solution was supplemented to the volume of 1 L with the buffer solution at room temperature, and after being left to stand for 2 h, it was ready for use.

### Experimental process:

An appropriate amount of each test substance was placed into separate 10 mL PE tubes, and FaSSIF (pH 6.5) buffer solution and FeSSIF (pH 5.0) buffer solution, each 10 mL, were added into the tubes, respectively. The tubes were then placed in a constant temperature water bath shaker and shaken at 37 °C for 24 h. After 24 h, samples were collected and centrifuged. The supernatants were diluted, and the solubility of the supernatants was measured.

### The experimental results are as follows:

**Table 4. Solubility of different crystal forms in test solutions with different pH values**

| Test substance | 24-hour solubility in buffer solutions with different pH values (mg/mL) | |
|---|---|---|
| | pH6.5 | pH5.0 |
| Compound of formula (I) | 0.0002 | 0.004 |
| Crystal form I | 0.052 | 0.29 |
| Crystal form II | 0.023 | 0.31 |
| Crystal form III | 0.023 | 0.025 |
| Crystal form IV | 0.023 | 0.31 |

Conclusion: Compared with the compound of formula (I), the solubility of the crystal forms I, II, III, and IV of the methanesulfonate of the compound in test solutions at pH 5.0 and pH 6.5 was significantly improved.

The above description is only for the purpose of illustrating preferred examples of the present disclosure, and is not intended to limit the scope of the present disclosure. Any modifications, equivalents, improvements, and the like made without departing from the spirit and principle of the present disclosure should be included in the protection scope of the present disclosure.

## Claims

1. A crystal form I of a methanesulfonate of compound N-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 8.13±0.2°, 14.78±0.2°, 21.82±0.2°, 22.24±0.2°, 23.91±0.2°, 24.24±0.2°, 25.19±0.2°, and 26.94±0.2°,

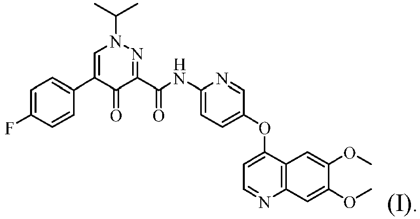

2. The crystal form I according to claim 1, further comprising characteristic peaks in an X-ray powder diffraction pattern using Cu-Kα radiation at 2θ angles of 11.11±0.2°, 17.37±0.2°, and 20.27±0.2°.

3. The crystal form I according to claim 2, further comprising characteristic peaks in an X-ray powder diffraction pattern using Cu-Kα radiation at 2θ angles of 12.52±0.2°, 13.75±0.2°, and 19.13±0.2°.

4. A preparation method for the crystal form I according to claim 1, comprising:
dissolving the compound of formula (I) in a single or mixed solvent, stirring until complete dissolution, then slowly adding methanesulfonic acid while stirring, and stirring until the crystal form I is precipitated, wherein
the single or mixed solvent is selected from one of or a mixture of two or more of isopropanol, methanol, ethanol, tetrahydrofuran, dichloromethane, 1,4-dioxane, toluene, acetone, ethyl acetate, acetonitrile, methyl tert-butyl ether, 2-methyltetrahydrofuran, dimethyl sulfoxide, and water; preferably, the single or mixed solvent is selected from methanol, ethanol, tetrahydrofuran, dichloromethane, 1,4-dioxane, and a mixed solution of isopropanol/ethanol.

5. A crystal form II of a methanesulfonate of compound *N*-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 12.66±0.2°, 13.68±0.2°, 15.74±0.2°, 18.57±0.2°, 21.16±0.2°, and 22.19±0.2°,

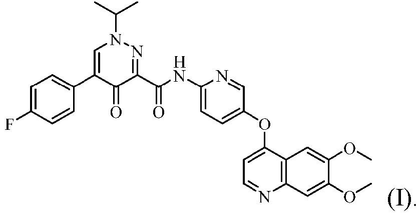

6. The crystal form II according to claim 5, further comprising characteristic peaks in an X-ray powder diffraction pattern using Cu-Kα radiation at 2θ angles of 18.09±0.2°, 20.13±0.2°, 24.88±0.2°, and 26.31±0.2°.

7. The crystal form II according to claim 6, further comprising characteristic peaks in an X-ray powder diffraction pattern using Cu-Kα at 2θ angles of 10.02±0.2°, 16.36±0.2°, and 29.41±0.2°.

8. A crystal form III of a methanesulfonate of compound *N*-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 10.32±0.2°, 10.58±0.2°, 12.55±0.2°, 17.30±0.2°, 21.49±0.2°, 21.95±0.2°, and 24.22±0.2°,

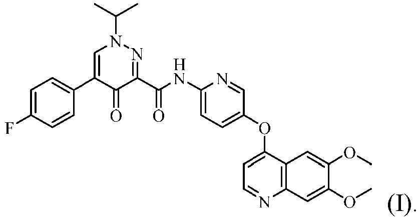

9. The crystal form III according to claim 8, further comprising characteristic peaks in an X-ray powder diffraction pattern using Cu-Kα radiation at 2θ angles of 9.47±0.2°, 13.04±0.2°, 20.66±0.2°, and 23.61±0.2°.

10. The crystal form III according to claim 9, further comprising characteristic peaks in an X-ray powder diffraction pattern using Cu-Kα radiation at 2θ angles of 8.61±0.2°, 15.34±0.2°, and 25.71±0.2°.

11. A crystal form IV of a methanesulfonate of compound *N*-(5-((6,7-dimethoxyquinolin-4-yl)oxy)pyridin-2-yl)-5-(4-fluorophenyl)-1-isopropyl-4-oxo-1,4-dihydropyridazine-3-carboxamide of formula (I), comprising characteristic peaks in an X-ray powder diffraction pattern using Cu-Kα radiation at 2θ angles of 9.71±0.2°, 12.66±0.2°, 17.59±0.2°, 19.35±0.2°, 22.01±0.2°, 23.40±0.2°, and 24.77±0.2°,

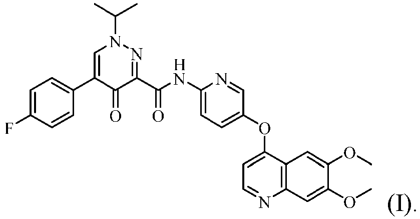

12. The crystal form IV according to claim 11, further comprising characteristic peaks in an X-ray powder diffraction pattern using Cu-Kα radiation at 2θ angles of 15.11±0.2°, 22.34±0.2°, and 23.92±0.2°.

13. The crystal form IV according to claim 12, further comprising characteristic peaks in an X-ray powder diffraction pattern using Cu-Kα radiation at 2θ angles of 10.26±0.2°, 11.78±0.2°, and 12.45±0.2°.

14. A pharmaceutical composition, comprising the crystal form I according to any one of claims 1-3, the crystal form II according to any one of claims 5-7, the crystal form III according to any one of claims 8-10 or the crystal form IV according to any one of claims 11-13, and one or more second therapeutically active agents.

15. A pharmaceutical formulation, comprising the crystal form I according to any one of claims 1-3, the crystal form II according to any one of claims 5-7, the crystal form III according to any one of claims 8-10 or the crystal form IV according to any one of claims 11-13, and one or more pharmaceutical carriers.

16. Use of the crystal form I according to any one of claims 1-3, the crystal form II according to any one of claims 5-7, the crystal form III according to any one of claims 8-10, the crystal form IV according to any one of claims 11-13, the pharmaceutical composition according to claim 14, or the pharmaceutical formulation according to claim 15 in the manufacture of a medicament for treating and/or preventing a related disease caused by an abnormal signaling pathway resulting from abnormal expression of a TAM family kinase receptor and/or CSF 1R kinase receptor and/or a ligand thereof.

17. Use of the crystal form I according to any one of claims 1-3, the crystal form II according to any one of claims 5-7, the crystal form III according to any one of claims 8-10, the crystal form IV according to any one of claims 11-13, the pharmaceutical composition according to claim 14, or the pharmaceutical formulation according to claim 15 in the manufacture of a medicament for treating and/or preventing an NTRK-caused related disease.
